(19) Europäisches Patentamt
European Patent Office
Office européen des brevets

(11) Veröffentlichungsnummer: **0 211 271**
**B1**

(12) # EUROPÄISCHE PATENTSCHRIFT

(45) Veröffentlichungstag der Patentschrift: **12.10.88**

(51) Int. Cl.⁴: **C 07 C 121/75**, C 07 C 120/04

(21) Anmeldenummer: **86109481.1**

(22) Anmeldetag: **11.07.86**

(54) **Verfahren zur Herstellung von Veratrylcyanid.**

(30) Priorität: **31.07.85 DE 3527338**

(43) Veröffentlichungstag der Anmeldung: **25.02.87 Patentblatt 87/9**

(45) Bekanntmachung des Hinweises auf die Patenterteilung: **12.10.88 Patentblatt 88/41**

(84) Benannte Vertragsstaaten: **BE CH DE FR GB IT LI NL SE**

(56) Entgegenhaltungen:
**DE-C-896 344**
**US-A-2 734 908**
**US-A-2 783 265**

(73) Patentinhaber: **BASF Aktiengesellschaft, Carl-Bosch- Strasse 38, D-6700 Ludwigshafen (DE)**

(72) Erfinder: **Müller, Josef, Dr., Rieslingstrasse 15, D-6711 Grosskarlbach (DE)**
Erfinder: **Wiersdorff, Walter- Wielant, Dr., Blockfeldstrasse 15, D-6704 Mutterstadt (DE)**
Erfinder: **Burst, Wolfram, Illerstrasse 7, D-6800 Mannheim 24 (DE)**
Erfinder: **Dralle, Heinz, Dr., Friederikenweg 8, D-4950 Minden (DE)**
Erfinder: **Schaeffner, Ernst, Dr., Hornacker 24, D-4950 Minden (DE)**
Erfinder: **Steinkamp, Rolf, Dr., Geminiweg 18, D-4950 Minden (DE)**

**EP 0 211 271 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1988

**Beschreibung**

Die Erfindung betrifft ein Verfahren zur Herstellung von Veratrylcyanid (VCN) wobei man die toluolische Lösung des durch Chlormethylierung von Veratrol erhaltenen Veratrylchlorids direkt unter Zusatz von Wasser, Keton und Katalysatoren mit Natriumcyaniden umsetzt.

Veratrylcyanid ist ein wichtiges Zwischenprodukt im pharmazeutischen Bereich und wird beispielsweise für die Herstellung von Verapamil und Papaverin verwendet.

Da einerseits die Chlormethylierung von Alkoxybenzolen wie Veratrol in Toluol als Lösungsmittel durchgeführt wird, andererseits die Weiterverarbeitung des erhaltenen Veratrylchlorids zum Veratrylcyanid in polaren Lösungsmitteln erfolgt, muß nach den bekannten Verfahren (US-PS-2 734 908, Arch. Pharm. 296, 591 (1963) ein Lösungsmittelwechsel vorgenommen werden. Abgesehen vom technischen und energetischen Aufwand hat die destillative Abtrennung des Toluols - bedingt durch die thermische Instabilität des Veratrylchlorids - Ausbeuteverluste in erheblichem Ausmaß zur Folge.

Die US-PS-2 734 908 beschreibt die Umsetzung von Veratrylchlorid mit Cyanid in Ketonen unter Zusatz von Alkaliiodiden. Diese Verfahrensweise ist jedoch nachteilig, da wegen der sehr langen Reaktionszeiten und niedrigen Konzentrationen geringe Raum-Zeit-Ausbeuten resultieren. Zur Aufarbeitung muß vom Salzrückstand abfiltriert, dann das Lösungsmittel abdestilliert und der Rückstand in einem zweiten Lösungsmittel gelöst werden. Dann wird das Gemisch fraktioniert destilliert. Diese Operationen sind technisch aufwendig und teuer.

Arch. Pharm. 294, 591 (1963) beschreibt die gleiche Umsetzung in Dimethylformamid unter Zusatz von 5 % $H_2O$ in 85 %-iger Ausbeute. Auch hier stellt sich das Problem der Salzabtrennung. Auf die Nebenproduktbildung nach diesem Verfahren wird in Chem. Pharm. Bull 19, 1374 (1971) hingewiesen.

Die deutsche Patentanmeldung P-3 341 306.1 beschreibt die Umsetzung von p-Methoxybenzylchlorid mit Cyanid in Methylisobutylketon mit Hilfe von Phasentransferkatalysatoren unter Zusatz von Wasser. Hier werden zwar gute Ausbeuten und Raum-Zeit-Ausbeuten erreicht, für die technische Synthese von VCN ist das Verfahren jedoch ebenfalls ungeeignet, da die Chlormethylierung von Veratrol in Methylisobutylketon mißlingt und, falls für die vorausgegangene Chlormethylierung von Veratrol das dafür übliche Solvens Toluol verwendet wurde, ein Lösungsmittelwechsel erforderlich wäre, der in erheblichem Maß eine Zersetzung des thermisch instabilen Veratrylchlorids zur Folge hätte.

Der Erfindung lag daher die Aufgabe zugrunde, ein technisches Verfahren zur Herstellung von Veratrylcyanid zu entwickeln, das die bisher bekannten Verfahren vereinfacht und in technischer und wirtschaftlicher Hinsicht verbessert.

Die Lösung dieser Aufgabe besteht in einem Verfahren zur Herstellung von Veratrylcyanid durch Chlormethylierung von Veratrol in toluolischer Lösung und anschließende Umsetzung des erhaltenen Veratrylchlorids mit 1 bis 5 Mol Alkalicyanid, wobei man das Toluol von der Chlormethylierung für die Umsetzung mit Cyanid nicht entfernt, und wobei man vor der Umsetzung mit Cyanid 3 bis 25 Gew.-%, bezogen auf Veratrylchlorid Wasser, 5 bis 50 Gew.-%, bezogen auf Toluol, eines Ketons mit 3 bis 6 Kohlenstoffatomen und bis 10 Gew.-%, bezogen auf Veratrylchlorid, eines Phasentransferkatalysators zusetzt.

Die Synthese lauft nach folgendem Schema ab:

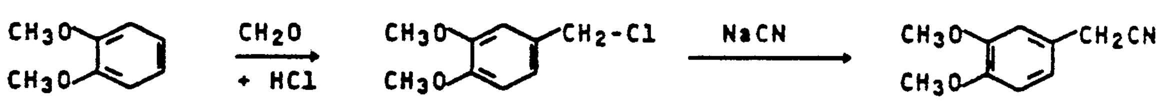


Die Chlormethylierung des Veratrols in Toluol erfolgt nach üblichen Methoden, wie sie z. B. im DDR-Patent 9451 sowie in Houben-Weyl, Methoden der org. Chem., 4. Aufl., Georg-Thieme-Verl., Stuttgart 1962, Bd. 5/3, S. 1001 beschrieben sind.

Die Umsetzung des Veratrylchlorids mit Cyanid kann wie folgt durchgeführt werden: Ein Gemisch von Toluol, Keton, Veratrylchlorid, Alkalicyanid, Phasentransferkatalysator und Wasser in den im Anspruch angegebenen Mengenverhältnissen wird während 1 bis 5 Stunden bei 50 bis 95°C gehalten. Im Vergleich zu den bekannten Verfahren liefert das erfindungsgemäße Verfahren Veratrylcyanid in besserer Ausbeute, Raum-Zeit-Ausbeute und Reinheit. Die Abtrennung des Endstoffs von den Salzen erfordert kein zusätzliches Solvens. Der Gehalt am unerwünschten Nebenprodukt Veratrylalkohol ist gering; dadurch gestaltet sich die Aufarbeitung, besonders die fraktionierte Destillation des Veratrylcyanidseinfach.

Ein weiterer wesentlicher Vorteil des erfindungsgemäßen Verfahrens besteht darin, daß man die aus der Chlormethylierung erhaltene toluolische Rohveratrylchloridlösung direkt, d. h. ohne Solvenswechsel, in die Cyanisierungsstufe einsetzen kann. Dies geschieht unter Zusatz von geringen Mengen eines Ketons (5 bis 50, vorzugsweise 7 bis 15 Gew.-%, bezogen auf Toluol), eines Phasentransferkatalysators (1 bis 10, vorzugsweise 1 bis 3 Gew.-% bezogen auf Verytrylchlorid) und von Wasser (3 bis 25, vorzugsweise 5 bis 10 Gew.-%, bezogen auf Veratrylchlorid).

Der Einsatz an Alkalicyanid beträgt 100 bis 500, vorzugsweise 110 bis 150 Mol.-%, bezogen auf Veratrylchlorid. Als Alkalicyanide sind Kaliumcyanid und insbesondere Natriumcyanid bevorzugt.

Als Ketone werden solche der Formel

$$R^1-\overset{\overset{\displaystyle O}{\|}}{C}-R^2,$$

worin $R^1$ Methyl oder Ethyl und $R^2$ einen Alkylrest mit 1 bis 4 Kohlenstoffatomen bedeuten, mit der Maßgabe, daß die Gesamtzahl der C-Atome in $R^1$ und $R^2$ 2 bis 5 beträgt, oder worin $R^1$ und $R^2$ zusammen mit der Carbonylgruppe auch einen 5- oder 6-gliedrigen Ring bezeichnen können, verwendet.

Als Ketone kommen z. B. in Frage: Methylethyl-, Diethyl-, Methylpropyl-, Methyl-i-butyl, Methyl-n-butyl-, Methyl-sec.-butyl-, Methyl-tert.-butylketon, Cyclopentanon, Cyclohexanon, bevorzugt Methylethylketon, Diethylketon, Cyclopentanon, insbesondere Aceton.

Unter Phasentransferkatalysatoren versteht man solche Katalysatoren, die den Transport von Stoffen in binären flüssigen Systemen (aus Wasser und organischem Lösungsmittel) verbessern. Schon kleine Mengen an Katalysator ergeben den Transfereffekt, d. h. die Ausgangsstoffe gelangen mit Hilfe kleiner Katalysatormengen von der einen in die andere Phase. Bezüglich der Definition, Herstellung und Eigenschaften solcher Phasentransferkatalysatoren wird z.B. auf J. Amer. Chem. Soc. 93, 195 (1971) und auf Chem. Education 55, 429, 350 (1978), verwiesen.

Als Phasentransferkatalysatoren kommen zweckmäßig quarternäre Salze in Betracht, insbesondere Katalysatoren der Formel

$$\left[\begin{array}{c} R^3 \\ | \\ R^2 - Y - R^4 \\ | \\ R^1 \end{array}\right]^{\oplus} \quad Z^{\ominus}$$

worin die einzelnen Reste $R^1$ bis $R^4$ gleich oder verschieden sein können und jeweils einen aliphatischen, cycloaliphatischen, aromatischen oder araliphatischen Rest bedeuten, Y ein Stickstoffatom oder Phosphoratom bezeichnet und Z für ein Säureanion steht. Bevorzugte Katalysatoren sind solche, in deren Formeln die einzelnen Reste $R^1$ bis $R^4$ gleich oder verschieden sein können und jeweils einen Alkylrest oder einen Hydroxyalkylrest mit jeweils 1 bis 18, insbesondere 1 bis 7 Kohlenstoffatomen, einen Cycloalkylrest mit 5 bis 8 Kohlenstoffatomen, einen Aralkylrest oder Alkylarylrest mit 7 bis 12 Kohlenstoffatomen oder einen Phenylrest bedeuten. Aber auch tert. Amine in Verbindung mit Alkyliodid können eingesetzt werden. Besonders bevorzugt ist das System Triethylamin/Natriumiodid im Molverhältnis 1 : 0,5 bis 1 : 1,5.

Es kommen z. B. als Phasentransferkatalysatoren in Frage: Tetramethyl-, Tetra-n-propyl-, Tetraisopropyl-, Tetra-n-butyl-, Tetraisobutyl-, Tetrasec.-butyl-, Tetra-tert.-butyl Tetrapentyl-, Tetrahexyl-, Tetra-n-heptyl-, Tetraoctyl-, Tetranonyl-, Tetradecyl-, Tetraethyl-, Tetraundecyl-, Tetradodecyl-ammoniumchlorid; Tetra-β-hydroxyethyl-ammoniumchlorid, Tetra-ω-hydroxypropyl-ammoniumchlorid, Tetra-β-hydroxypropyl-ammoniumchlorid, Tetra-γ-hydroxybutyl-ammoniumchlorid Tetra-ω-hydroxybutyl- ammoniumchlorid, Tetra-β-hydroxybutyl-ammoniumchlorid, Tetra-(α,α-dimetyl-β-hydroxyethyl-ammoniumchlorid, Tetra-(α-methyl-β-hydroxypropyl)- ammoniumchlorid, Tetra-(β,β-dimethyl-β-hydroxyethyl)-ammoniumchlorid, Tetra-(α-ethyl-β-hydroxyethyl)-ammoniumchlorid, Tetra-(α-methyl-ω-hydroxypropyl)-ammoniumchlorid, Tetra-(β-methyl-ω-hydroxypropyl)-ammoniumchlorid, Tetra-β-hydroxypentyl-ammoniumchlorid, Tetra-ω-hydroxypentyl-ammoniumchlorid, Tetra-β-hydroxypentyl-ammoniumchlorid, Tetra-ω-hydroxypentyl-ammoniumchlorid, Tetra-γ-hydroxypentyl-ammoniumchlorid; durch quartäre Substituierung mit vorgenannten Substituenten und/oder mit der Phenyl-, Benzyl-, Cyclohexyl-, Toluyl-, Methylcyclohexyl-, Phenylethyl-, Phenylpropyl-, Phenylbutylgruppe am Stickstoffatom sich entsprechend aus Anilin, Benzylamin, o,m,p-Toluidin, Triphenyl-, Tribenzyl-, Tricyclohexyl-, Tri(methylcyclohexyl)-, Tri(phenylethyl)-, Tri(phenylpropyl)-, Tri(phenylbutyl)-amin in 2-, 3- und 4-Stellung einfach oder in 2,4-, 2,3-, 2,6-, 2,5-, 3,4-, 3,5-Stellung zweifach durch die Methylgruppe an jedem Phenylring subtituiertem Triphenylamin ergebende Ammoniumchloride, die auch Ammoniumchloride mit 4 vorgenannten, aber völlig oder teilweise unterschiedlichen Resten sein können, z. B. die sich durch Substituierung mit dem Methylrest aus N,N-Dimethylanilin, N-Methyl-N,N-diethylamin, N,N-Dicyclohexyl-n-methylamin, N-Methyl-N-ethyl-N-npropylamin ergebenden quartären Ammoniumchloride oder z. B. Dimethylbenzyldodecyl-, Cetyltrimethyl-, Methyltriethyl-, Dimethyldiphenyl-, Trimethyl-(o-tert.-butylphenyl)-, Triethyldodecyl-, Trimethyl-tridecyl-, Trimethyl-diphenylmethyl-, Trimethyl-N-dodecyl- Trimethyl-β-hydroxyethyl- N-Propyl-trimethyl-, Isoamyltrimethyl-, Benzyl-dimethyl-n-octyl-, Benzyl-trimethyl-, Benzyltriethyl-, Phenyl-trimethyl-, Dimethyl-dodecyl-phenyl-, Trimethyl-(phenyl (1)-ethyl)-, Trimethyl-(phenyl-(2)-ethyl)-ammoniumchlorid, entsprechende Ammoniumbromide; homologe quartäre Ammoniumsalze von anorganischen oder organischen einbasischen oder mehrbasischen Säuren wie Chlorwasserstoff, Bromwasserstoff, Iodwasserstoff, Perchlorsäure, Schwefelsäure, Phosphorsäure, salpetrige Säure, Salpetersäure, Kohlensäure; Sulfonsäuren wie Benzol- und p-Toluolsulfonsäure; Bor enthaltenden Säuren wie Borsäure, Borfluorwasserstoffsäure; oder entsprechende Gemische. Bevorzugt sind die quaternären Ammoniumsalze der Salzsäure, Bromwasserstoffsäure, Schwefelsäure, Phosphorsäure mit vorgenannten

Substituenten am Heteroatom und/oder Säureanionen sowie homologe Phosphoniumsalze.

Die Menge an Phasentransferkatalysator, der bei der Cyanisierung verwendet wird, beträgt 0,1 bis 10, vorzugsweise 1 bis 3 Gew.-%, bezogen auf eingesetztes Veratrylchlorid. Die Reaktion kann kontinuierlich oder diskontinuierlich bei 50 bis 95°C durchgeführt werden; besonders bevorzugt ist der Bereich zwischen 80 und 88°C (am Rückfluß).

Die Reaktionszeit beträgt 1,0 bis 5, vorzugsweise 1,5 bis 2,5 Stunden. Das ist wesentlich kürzer als nach den bisher bekannten Verfahren, wo die Gesamtzeit für Lösungsmittelwechsel und Umsetzung im Bereich von 10 bis 40 Stunden liegt. Das Ende der Reaktion erkennt man durch z. B. Dünnschicht- oder gaschromatographische Analyse.

Aus dem Reaktionsgemisch kann der Endstoff in üblicher Weise, z. B. durch Versetzen mit Wasser, Phasentrennung und Destillation, abgetrennt werden.

Die in den Beispielen genannten Teile beziehen sich auf das Gewicht.

**Beispiel 1**

87 Teile Toluol, 2,4 Teile Wasser, 18,65 Teile Veratrylchlorid, 0,11 Teile NaI, 0,12 Teile Triethylamin, 6 Teile NaCH und 10 Teile Aceton wurden 3,5 Stunden bei 85°C gerührt. Dann wurden 20 Teile Wasser zugefügt, 5 Minuten gerührt und die Phasen getrennt. Nach Abdestillieren des Lösungsmittels wurde der Rückstand im Vakuum destilliert. Bei 162 bis 164°C und 6 mbar gingen 16,9 Teile (= 95 %) VCN über.

Es waren 0,9 Gew.-%, bezogen auf Destillat, Veratrylalkohol im Destillat enthalten.

**Beispiel 2**

Es wurde wie bei Beispiel 1 verfahren, die Toluolmenge jedoch auf 17 Teile reduziert. Die Reaktionszeit betrug 1,5 Stunden. Es wurden 17,2 Teile (= 97 %) VCN erhalten.

0,8 % Veratrylalkohol befanden sich im Destillat.

**Beispiel 3**

Es wurde wie bei Beispiel 1 verfahren, die Toluolmenge betrug 44 Teile, die Wassermenge 1,8 Teile, die Acetonmenge 5 Teile. Die Reaktionszeit war 2,5 Stunden. Es wurden 16,8 Teile (= 95 %) VCN erhalten.

1,2 % Veratrylalkohol waren im Destillat enthalten.

**Beispiel 4**

1650 Teile einer toluolischen Rohveratrylchloridlösung, enthaltend 510 Teile Veratrylchlorid, erhalten durch Chlormethylierung von Veratrol, wurden innerhalb von 15 Minuten zu einer auf 65°C vorgewärmten Mischung aus 305 Teilen Toluol, 140 Teilen Aceton, 197 Teilen NaCH, 3,1 Teilen NaI, 25 Teilen $H_2O$ und 5,2 Teilen Triethylamin gegeben. Nach der Zugabe wurde noch 1,5 Stunden bei 85°C gerührt, dann die Mischung mit 700 Teilen $H_2O$ versetzt, die untere wäßrige Phase abgetrennt und die organische Phase ein zweites Mal mit 200 Teilen $H_2O$ nachgewaschen. Die organische Phase wurde vom Toluol befreit und der Rückstand über eine Kolonne bei 6 mbar und 162 bis 164°C destilliert. Es wurden 445 Teile (= 92 %) VCN erhalten.

1,6 % Veratrylalkohol waren im Destillat enthalten.

**Vergleichsbeispiel ohne Ketonzusatz**

87 Teile Toluol, 20 Teile $H_2O$, 6 Teile NaCH, 0,11 Teile NaI, 0,12 Teile Triethylamin und 18,65 Teile Veratrylchlorid wurden 4 Stunden bei 85°C gerührt. Nach Abtrennen der wäßrigen Phase wurde die organische Phase in der üblichen Weise aufgearbeitet. Es wurden 16,7 Teile (= 87,5 %) VCN erhalten.

7,2 % Veratrylalkohol waren im Destillat enthalten.

**Patentanspruch**

Verfahren zur Herstellung von Veratrylcyanid durch Chlormethylierung von Veratrol in toluolischer Lösung und anschließende Umsetzung des erhaltenen Veratrylchlorids mit 1 bis 5 Mol Alkalicyanid, dadurch gekennzeichnet, daß man das Toluol von der Chlormethylierung für die Umsetzung mit Cyanid nicht entfernt und daß man vor der Umsetzung mit Cyanid 3 bis 25 Gew.-%, bezogen auf Veratrylchlorid, Wasser, 5 bis 50 Gew.-%, bezogen auf Toluol, eines Ketons mit 3 bis 6 Kohlenstoffatomen, und 0,1 bis 10 Gew.-%, bezogen auf Veratrylchlorid, eines Phasentransferkatalysators zusetzt.

**Claim**

A process for the preparation of veratryl cyanide by chloromethylating veratrol in solution in toluene and then reacting the resulting veratryl chloride with from 1 to 5 moles of an alkali metal cyanide, wherein the toluene from the chloromethylation is not removed for the reaction with cyanide, and from 3 to 25 % by weight, based on veratryl chloride, of water, from 5 to 50 % by weight, based on toluene, of a ketone of 3 to 6 carbon atoms and from 0.1 to 10 % by weight, based on veratryl chloride, of a phase transfer catalyst are added before the reaction with cyanide.

**Revendications**

Procédé pour la préparation de cyanure de veratryle par chlorométhylation de varatrol en solution toluénique, suivie de la réaction du chlorure de veratryle avec 1 à 5 moles de cyanure alcalin, caractérisé par le fait que, pour la réaction avec le cyanure, on n'élimine pas le toluène de la chlorométhylation, et qu'avant la réaction avec le cyanure, on ajoute 3 à 25 % en poids, rapportés au chlorure de veratryle, d'eau, 5 à 50 % en poids, rapportés en toluène, d'une cétone de 3 à 6 atomes de carbone, et 0,1 à 10 % en poids, rapportés au chlorure de veratryle, d'un catalyseur de transfert de phases.